# EUROPEAN PATENT APPLICATION

(11) **EP 3 064 945 A1**
(43) Date of publication of application: **07.09.2016**
(21) Application number: 16166503.9
(22) Date of filing: 22.02.2013
(51) Int. Cl.: G01N 33/574, A61N 5/10, A61K 51/10

(54) **ELEVATED PSMA IDENTIFIES LETHAL PROSTATE CANCERS BACKGROUND**

(30) Priority: 24.02.2012 US 201261602900 P
(62) Divisional of application: 13752566.3
(71) Applicant: Cornell University, Ithaca, NY 14850 (US)
(72) Inventor: Bander, Neil H., Chappaqua, NY New York (US)
(74) Representative: Williams, Andrea

(57) **Abstract**

Prostate cancer (PC) is an entity that encompasses different types of tumors, including adenocarcinomas and non-adenocarcinomas. Adenocarcinomas and non-adenocarcinomas respond to, and therefore should be treated, with different treatments. Even within the adenocarcinomas, the lethality of tumors is highly variable, from low risk/indolent/non-life threatening to high risk/lethal. The inability to accurately predict the behavior of a particular cancer makes treatment decisions difficult and highly inexact. Elevated expression of prostate specific membrane antigen (PSMA) expression is a molecular hallmark of lethal prostate adenocarcinoma. Assessment of PSMA expression levels to predict the behavior of a particular cancer will be useful in the diagnosis and treatment of PC.

## Description

### BACKGROUND

Prostate cancer (PC) is an entity that encompasses different types of tumors (adenocarcinomas (95%) and non-adenocarcinomas (5%)). Adenocarcinomas and non-adenocarcinomas respond to, and therefore should be treated with, different treatments. Even within the adenocarcinomas, the lethality of the tumors is highly variable, from low rislc/indolent/non-life-threatening to high risk/lethal; the latter category make PC the third most common cause of cancer deaths in men. The current inability to predict the behavior of a particular patient's cancer renders treatment decisions difficult, and highly inexact. It leads to over-treatment of the large number of patients whose PC is not life-threatening. Those over-treated patients suffer compromise in their quality of life from treatment that provides no benefit to that patient. Conversely, if a patient who has a lethal form of prostate cancer is under-treated in an effort to avoid side effects, the end result may be death. What is needed is a way to distinguish adenocarcinomas from non-adenocarcinomas and lethal from indolent PC. Ideally, this could be done non-invasively and could survey the entire prostate gland and/or the entire body. This would be in contrast with the current prostate biopsy approach which is invasive, entails complications of its own, and samples only a very small part of the entire prostate gland.

### BRIEF SUMMARY OF THE INVENTION

### PSMA is a Biomarker for Prostate Adenocarcinomas

The expression of PSMA is tightly linked to that of the androgen receptor (AR). Prostate adenocarcinomas are AR⁺/PSMA⁺; non-adenocarcinoma variants (e.g., small cell, neuroendocrine, sarcoma, *etc*.) make up a minority (≈5%) of all prostate cancers and have a phenotype of AR-/PSMA-. Somewhat commonly, non-adenocarcinomas derive from adenocarcinomas and these can manifest a mixed phenotype including both AR⁺/PSMA⁺ and AR⁻/PSMA⁻ cells. While the backbone of treatment of adenocarcinomas is hormonal therapy followed by taxane-based chemotherapy, non-adenocarcinomas do *not* respond to hormonal therapy and are treated instead with platinum-based chemotherapy.

### High PSMA is a Molecular Hallmark of Lethal Prostate Adenocarcinoma

Archived biopsy or surgical specimens from patients obtained years earlier at the time of their diagnosis were obtained to measure PSMA levels in prostate cancers (PC). Surprisingly, in patients with lethal prostate cancer, 63 of 66 (95%) patients with lethal PC had very high levels of PSMA at the time of diagnosis-years before developing metastatic or castrate-resistant PC (CRPC). This result was surprising because it has been long thought that PSMA levels begin relatively low and increase over time as the disease progresses-*i.e*., with respect to PSMA levels, localized PC < metastatic PC < hormone-refractory PC. That PSMA was very high early in the course of disease in those patients who later develop metastatic, lethal PC was not anticipated.

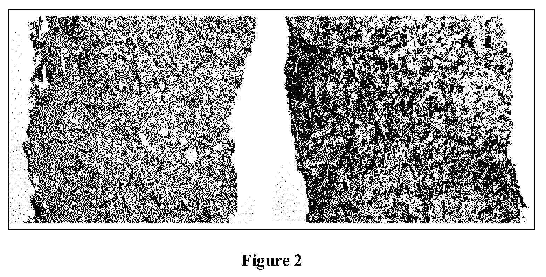

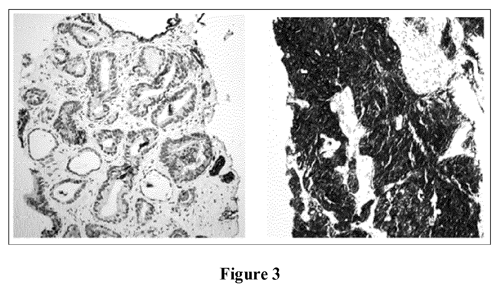

This association of high PSMA and lethal PC was further reinforced by the finding in several patients who had both histological Gleason pattern (associated with low risk, indolent PC) as well as Gleason pattern 4 or 5 (high risk, lethal PC): the Gleason pattern 3 (low risk, indolent PC) foci all had low PSMA expression, whereas the Gleason 4 or 5 lesions (high risk, lethal PC) were uniformly strongly positive for PSMA (*see* Figures 2 and 3). This is a telling observation. That is, within the same individual, the same genetic background, the same prostate, and the same hormonal milieu, the clinically benign pattern 3 is easily distinguished from the significantly more lethal histology simply by virtue of PSMA expression. It is also consistent with several previous publications showing that higher PSMA expression measured in radical prostatectomy specimens predicted a higher tumor recurrence rate (Ross, et al., Clin Cancer Res 2003; 9, 6357-62; Perner, et al., Hum Pathol 2007;38: 696-701; and Minner, et al., Prostate. 2011; 71:281-288). Examining biopsies from patients with indolent PC that did not progress or lead to the death of the patient, showed these lesions were only weak or negative for PSMA expression. The forgoing indicates that PSMA is a molecular hallmark of lethal prostate adenocarcinoma.

The ability to distinguish lethal PC from indolent PC has been a "Holy Grail" in PC. PC is the third most common cause of cancer deaths in American men. It is diagnosed in approximately 240,000 men annually in the U.S., with about 28,000 deaths yearly due to PC. However, the rate of diagnosis is far higher than the rate of death. It is well known that the substantial majority of patients diagnosed with prostate cancer will not die because of their PC. Since it has been impossible to distinguish those patients with "indolent" PC from those whose disease may be lethal, almost everyone undergoes treatment with radical surgery and/or radiation treatments. Those treatments, while intended to prevent metastasis and death due to PC, unfortunately also compromise quality of life. While this compromise may be acceptable in cases where it prevents death, it is not acceptable in cases when the patient was not at risk of death. Unfortunately, identifying patients who are at risk of metastasis and death versus those who are not, has been an unfulfilled goal to date. Therefore, virtually all diagnosed patients must endure the side effects even though only a minority actually benefit. However, if one could do a simple test to distinguish the lethal from the indolent form of the disease, treatment (or lack thereof) could be tailored to the patient: the patient with a lethal form of the disease could be advised to have surgery and/or radiation, whereas the person with indolent disease could be monitored.

Testing shows high PSMA expression identifies those patients with lethal PC. Testing can be performed on tissue specimens (*e.g*., prostate biopsy, TURP, prostatectomy tissue), on bodily fluids (*e.g.,* blood, urine, prostatic fluid, semen), or by imaging (*e.g.,* by any agent capable of specific binding to PSMA such as: antibodies, antibody derivatives, PSMA ligands, small molecule PSMA binders, PSMA enzyme inhibitors, PSMA-binding peptides, PSMA-binding aptamers, *etc*)*.* These PSMA binding agents can be labeled with any agent that allows detection (*e.g*., radioisotopes such as Zirconium-89, Technetium-99m, radioiodine, Indium-111, Copper-64, Iodine-124, *etc*.; or dyes such as indocyanine green, cyanine dyes, fluorescent dyes, infrared dyes, enzymes, *etc*). Imaging may be performed using positron emission tomography (PET), PET/Computed tomography (CT), PET/Magnetic resonance (MR), planar imaging, SPECT imaging, optical imaging, or dye imaging, *etc.*

Such approaches have diagnostic and therapeutic uses and value. Diagnostically, one could (non-invasively) image a patient's prostate to determine if it harbored a lethal cancer and, if it did, pinpoint the location for a directed confirmatory biopsy (rather than the dozen or more rather random sampling biopsies currently utilized). If a lethal lesion is not identified on the imaging study, a biopsy can be omitted.

Instead of subjecting some 1,000,000 men in the U.S. annually to prostate biopsy (as is currently practiced), only those patients with potentially lethal lesions would undergo a (directed) biopsy, reducing the biopsy rate by up to more than 90%, complications by up to more than 99% and health care costs by up to more than $1.5 billion.

In families with a history of PC, where there is increased risk for other male family members to develop PC, those family members could be imaged to detect the presence of any lesions. Such screening could be carried out annually or at other appropriate intervals to monitor for development of a lethal lesion or lesions.

For patients who have a biopsy showing low grade/indolent PC, an imaging study can screen the whole gland to look for a possible occult lethal PC. For patients diagnosed with low grade/indolent PC, the ability to sensitively, specifically, effectively and non-invasively image the prostate would provide a dramatically improved surveillance tool. Currently, these patients who undergo "active surveillance," are subjected to an annual biopsy consisting of 12 or more needle sticks into the prostate to determine if a higher risk/lethal lesion has developed that would require more aggressive treatment.

A molecular biomarker capable of distinguishing prostate adenocarcinoma from a non-adenocarcinoma can guide therapy. The backbone of adenocarcinoma treatment is hormonal therapy with or without other treatments (*e.g*., taxane-based chemotherapy, immunotherapy, *etc*); non-adenocarcinomas would not be treated with hormonal therapy and use different chemotherapy agents (*e.g.,* cisplatin, etoposide, *etc*.)*.*

A molecular biomarker capable of distinguishing lethal from indolent PC could spare millions of men from interventions that unnecessarily compromise their quality of life and cost the health care system needless billions of dollars. It would allow treatment to be tailored to the underlying risk of the cancer. Those cancers that are indolent in nature could simply be monitored; those cancers with lethal risk could be treated more aggressively with surgery or radiation. Image mapping of the site/s of lesions could guide use of focal ablation therapies within the prostate.

PSMA imaging of metastatic sites would aid in determining the location of where biopsies could be taken. This will improve the yield of biopsies, currently at only 25%. PSMA imaging would also provide a prostate cancer-specific imaging tool to evaluate response to drug therapy. Currently no such imaging modality exists. Computed tomography (CT) and magnetic resonance (MR) merely show masses, which may be either benign or malignant. Even in the case of a malignancy, such malignancy may be prostate cancer or another malignancy. Bone scans merely reflect bone metabolism and do not directly image the tumor. As a result, increased uptake on bone scan may be tumor or a benign process (e.g., inflammation, fracture). Even in the case of tumor improving from treatment, the bone scan often yields a paradoxical result: getting worse due to healing of bone. A PC-specific scan, however, would more accurately guide treatment-patients with improving scans would be maintained on the therapy; patients whose scans show progression of disease could be changed to another, possibly more beneficial, treatment. Evaluation of new treatments or new combinations of treatments would be improved by application of a tumor-directed imaging study. This would aid development of new treatments

### BRIEF DESCRIPTION OF THE DRAWINGS

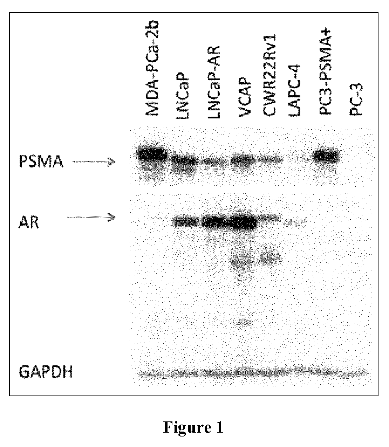

Figure 1 shows expression of PSMA and AR by cell lines. Equal amounts of cell lysates were loaded in each lane. PSMA was detected by monoclonal antibody (mAb) J591; AR was detected by mAb anti-AR (AR441). GAPDH was used as a loading control. Six of the cell lines (MDA-PCa-2b, LNCaP, LNCaP-AR, VCAP, CWR22Rv1 and LAPC-4) expressed both AR and PSMA. MDA-PCa-2b expresses a relatively low level of AR, just barely visible at this exposure. CWR22Rv1 expresses a slightly larger AR protein (114 KD, versus 110 KD) due to duplication of exon 3). PC3 and another cell line, DU145 (not shown), were AR⁻/PSMA⁻. PC3-PSMA is the PC3 line transfected with PSMA.
Figure 2 shows two needle core biopsies from the same patient's prostate. The biopsy on left shows a Gleason pattern 3; on the right is an area of pattern 4 or 5. Both are immunohistochemically stained for PSMA. The low grade lesion barely expression PSMA; the high grade lesion is strongly PSMA-positive.
Figure 3 also shows two needle core biopsies from another patient's prostate. The biopsy on left shows an area of Gleason pattern 3; on the right is an area of pattern 5. Both are immunohistochemically stained for PSMA. The low grade lesion is PSMA-negative; the high grade lesion is strongly PSMA-positive.

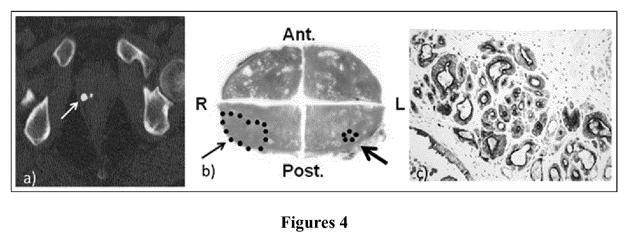

Figure 4 shows that PSMA expression reveals tumor site, confirmed by pathology and immunohistochemistry. Panel (a) shows a ⁸⁹Zr-J591 pre-surgical PET scan with high intensity focal uptake in the right apex (white arrow). Panel (b) shows an H&E stained specimen, showing a Gleason 7 prostate cancer legion in the right apex (thin arrow) corresponding to the focus shown in Panel (a); and a small focus of Gleason 6 (thick arrow), which was not shown in the PET scan. Panel (c) shows a high magnification immunohistochemical image of the right sided lesion in Panel (b), demonstrating positive staining for PSMA. These data demonstrate that an anti-PSMA antibody (e.g., ⁸⁹Zr-J591), for imaging, can be used to effectively visualize intraprostatic cancer foci and may preferentially identify lesions with Gleason sum ≥ 7, while missing lesions with Gleason sum ≤ 6.

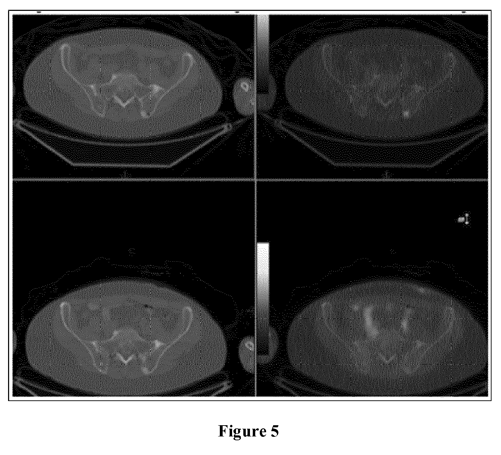

Figure 5 shows PET and CT scans from a patient with metastatic prostate cancer. These panels show CT scans (left column) and PET scans (right column). The upper right panel shows a ⁸⁹Zr-J591 image, which shows high uptake in the left iliac bone, highlighting a bone metasis, which is also observed by CT. However, this is not observed in the Fludeoxyglucose (18F) PET (lower right panel).

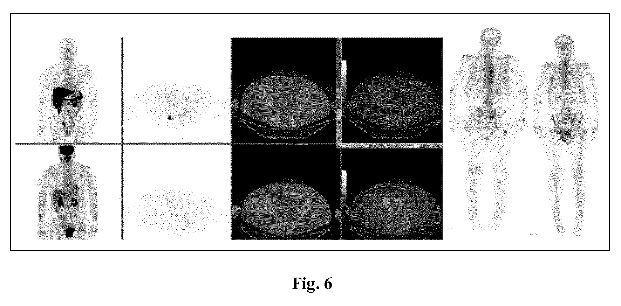

Figure 6 shows PET, CT, and bone scans from a patient with prostate carcinoma. The left panel show PET scans (with the upper panels showing 89Zr-J591 imaging and the lower panels showing FDG imaging); the middle panel show CT scans (with the upper panels showing 89Zr-J591 imaging and the lower panels showing FDG imaging); and the right panel shows bone scans. These data show that imaging using an anti-PSMA antibody, ⁸⁹Zr-J591, shows more intense uptake into the lesion, as compared to the FDG PET scanning. Additionally, these data show involvement of the skeletal system with uptake in a number of lesions that were not clearly seen on bone scan and that were also non-FDG avid.

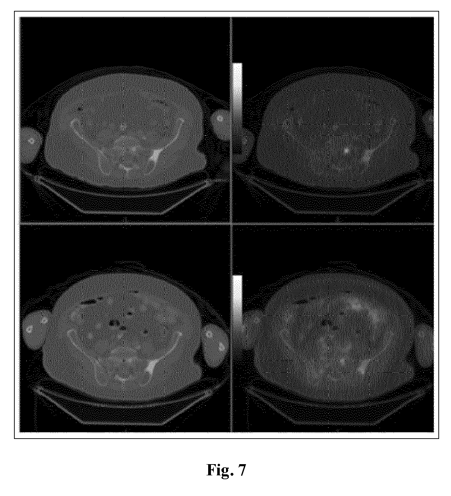

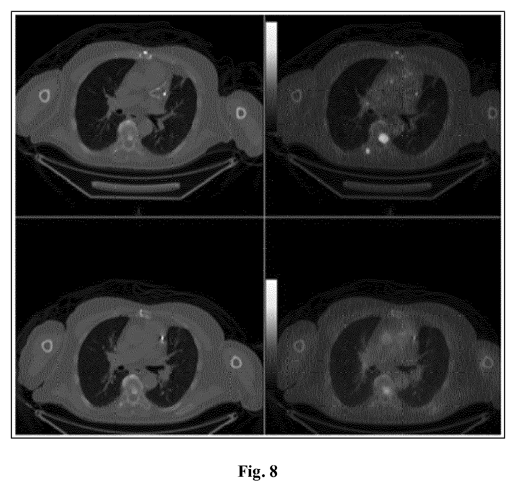

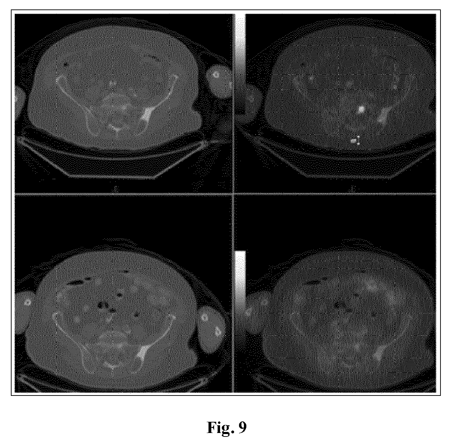

Figures 7-9 show CT and PET/CT scan taken from the same patient as from Figure 6. In these figures, the left panel shows CT scans (with the upper panels showing 89Zr-J591 imaging and the lower panels showing FDG imaging) and the right panel shows PET/CT scans (with the upper panels showing 89Zr-J591 imaging and the lower panels showing FDG imaging. Taken together, these data show that PSMA PET is superior to FDG PET at showing prostate cancer lesions. In all cases, the lesions were biopsied and confirmed to be prostate cancer (data not shown).

### EXAMPLES

### PSMA and AR Expression by Cell Lines

Expression of PSMA and AR were assessed by cell lines. Figure 1 shows expression of PSMA and AR by cell lines. Equal amounts of cell lysates were loaded in each lane. PSMA was detected by monoclonal antibody (mAb) J591; AR was detected by mAb anti-AR (AR441). GAPDH was used as a loading control. Six of the cell lines (MDA-PCa-2b, LNCaP, LNCaP-AR, VCAP, CWR22Rv1 and LAPC-4) expressed both AR and PSMA. MDA-PCa-2b expresses a relatively low level of AR, just barely visible at this exposure. CWR22Rv1 expresses a slightly larger AR protein (114 KD, versus 110 KD) due to duplication of exon 3). PC3 and another cell line, DU145 (not shown), were AR⁻/PSMA⁻. PC3-PSMA is the PC3 line transfected with PSMA.

### Immunohistochemical Staining for PSMA in Prostate Samples

Two needle core biopsies were obtained from two different patients (Figures 2 and 3). Each was stained immunohistochemically for PSMA expression. In Figure 2, the biopsy on left shows a Gleason pattern 3; on the right is an area of pattern 4 or 5 and the low grade lesion barely expression PSMA; the high grade lesion is strongly PSMA-positive. In Figure 3, the biopsy on left shows an area of Gleason pattern 3; on the right is an area of pattern 5 and the low grade lesion is PSMA-negative; the high grade lesion is strongly PSMA-positive.

### PSMA Expression by PET, CT, and Bone Scanning

PSMA expression was assessed by PET, CT, and bone scanning (Figures 4-9). Figure 4 shows that PSMA expression reveals tumor site, confirmed by pathology and immunohistochemistry. Panel (a) shows a ⁸⁹Zr-J591 pre-surgical PET scan with high intensity focal uptake in the right apex (white arrow). Panel (b) shows an H&E stained specimen, showing a Gleason 7 prostate cancer legion in the right apex (thin arrow) corresponding to the focus shown in Panel (a); and a small focus of Gleason 6 (thick arrow), which was not shown in the PET scan. Panel (c) shows a high magnification immunohistochemical image of the right sided lesion in Panel (b), demonstrating positive staining for PSMA. These data demonstrate that an anti-PSMA antibody (*e.g*., ⁸⁹Zr-J591), for imaging, can be used to effectively visualize intraprostatic cancer foci and may preferentially identify lesions with Gleason sum ≥ 7, while missing lesions with Gleason sum ≤ 6.

Figure 5 shows PET and CT scans from a patient with metastatic prostate cancer. These panels show CT scans (left column) and PET scans (right column). The upper right panel shows a ⁸⁹Zr-J591 image, which shows high uptake in the left iliac bone, highlighting a bone metasis, which is also observed by CT. However, this is not observed in the Fludeoxyglucose (18F) PET (lower right panel).

Figure 6 shows PET, CT, and bone scans from a patient with prostate carcinoma. The left panel show PET scans (with the upper panels showing 89Zr-J591 imaging and the lower panels showing FDG imaging); the middle panel show CT scans (with the upper panels showing 89Zr-J591 imaging and the lower panels showing FDG imaging); and the right panel shows bone scans. These data show that imaging using an anti-PSMA antibody, ⁸⁹Zr-J591, shows more intense uptake into the lesion, as compared to the FDG PET scanning. Additionally, these data show involvement of the skeletal system with uptake in a number of lesions that were not clearly seen on bone scan and that were also non-FDG avid.

Figures 7-9 show CT and PET/CT scan taken from the same patient as from Figure 6. In these figures, the left panel shows CT scans (with the upper panels showing 89Zr-J591 imaging and the lower panels showing FDG imaging) and the right panel shows PET/CT scans (with the upper panels showing 89Zr-J591 imaging and the lower panels showing FDG imaging. Taken together, these data show that PSMA PET is superior to FDG PET at showing prostate cancer lesions. In all cases, the lesions were biopsied and confirmed to be prostate cancer (data not shown).

### PSMA Expression As a Prognostic Tool for Overall Survival

A non-invasive means of measuring PSMA expression with radiolabeled J591 imaging was tested to assess its capacity as a prognostic tool for overall survival in patients with metastatic castration resistance prostate cancer.

Planar gamma camera images following infusion with radiolabeled J591 (¹¹¹In-J591 or ¹⁷⁷Lu-J591) were semi-quantitatively scored using 2 methods by 2 independent radiologists blinded to outcome. A 5-point visual score (VS) of 0-4+ was assigned. In addition, Tissue Targeting Index (TTI), a novel metric designed to semi-quantitatively score images was calculated using the ratio of lesion count density (corrected for background) to whole body count density, with maximum (TTIₘₐₓ) and mean (TTIₐᵥₑ) scores recorded. Follow up, tabulating subsequent therapies and overall survival, was recorded and imaging scores were associated with overall survival using Cox regression analysis.

In this study, 130 men with metastatic castration resistance prostate cancer underwent radiolabeled J591 imaging. 86.2% had bone metastases, 51.5% lymph node, 16.9% lung, 9.2% liver. 87.7% had accurate targeting of known sites of disease by planar imaging. CALGB (Halabi) nomogram scores were prognostic for the population. As continuous variables, TTIₘₐₓ (p=0.013) and TTIₐᵥₑ (p=0.002) were associated with worse survival. VS demonstrated a trend for worse survival (p=0.09). In multivariate analysis, TTI maintained independent prognostic value when controlling for Halabi score (TTIₘₐₓ p=0.02, TTIₐᵥₑ p=0.004).

These studies demonstrate that the level of PSMA expression is associated with overall survival in men with metastatic castration resistance prostate cancer. Non-invasive measurements of PSMA expression may be semi-quantitatively measured with planar gamma-camera imaging following radiolabeled J591. High PSMA expression appears to indicate more aggressive tumor biology with increased castration-resistance.

### DESCRIPTION OF THE PREFERRED EMBODIMENTS

One aspect of the technology is a method of distinguishing between adenocarcinomas and non-adenocarcinomas in a prostate cancer patient comprising the steps of:
(a) assaying a patient's prostate specific membrane antigen (PSMA) expression; and (b) determining, from the assay, if the patient's PSMA expression is indicative of prostate cancer adenocarcinoma or non-adenocarcinoma. In a related aspect, the PSMA expression is assayed on tissue specimen, such as a prostate biopsy, transurethral resection of the prostate, or prostatectomy tissue. In yet another aspect, the PSMA expression is assayed on bodily fluids, such as blood, urine, prostatic fluid, or semen. In another aspect of the technology, the PSMA expression is assayed by imaging. In a related aspect, the imaging is conducted by employing any agent capable of specific binding to PSMA, such as an antibody, antibody derivative, PSMA ligand, small molecule PSMA binder, PSMA enzyme inhibitor, PSMA-binding peptide, or PSMA-binding aptamer. In a related aspect, the agent is labeled with another agent that allows detection, such as Zirconium-89, Technetium-99m, radioiodine, Indium-111, Copper-64, Iodine-124. In a parallel aspect, the agent is a dye, such as indocyanine green, cyanine dyes, fluorescent dyes, infrared dyes, or enzymes. In one aspect of the technology, the imaging is done by positron emission tomography (PET), PET/Computed tomography (CT), PET/Magnetic resonance (MR), planar imaging, SPECT imaging, optical imaging, or dye imaging.

One aspect of the technology is a method of distinguishing between lethal and indolent prostate cancer in a patient comprising the steps of: (a) assaying a patient's prostate specific membrane antigen (PSMA) expression; and (b) determining, from the assay, if the patient's PSMA expression is indicative of lethal or indolent prostate cancer. In a related aspect, the PSMA expression is assayed on tissue specimen, such as a prostate biopsy, transurethral resection of the prostate, or prostatectomy tissue. In yet another aspect, the PSMA expression is assayed on bodily fluids, such as blood, urine, prostatic fluid, or semen. In another aspect of the technology, the PSMA expression is assayed by imaging. In a related aspect, the imaging is conducted by employing any agent capable of specific binding to PSMA, such as an antibody, antibody derivative, PSMA ligand, small molecule PSMA binder, PSMA enzyme inhibitor, PSMA-binding peptide, or PSMA-binding aptamer. In a related aspect, the agent is labeled with another agent that allows detection, such as Zirconium-89, Technetium-99m, radioiodine, Indium-111, Copper-64, Iodine-124. In a parallel aspect, the agent is a dye, such as indocyanine green, cyanine dyes, fluorescent dyes, infrared dyes, or enzymes. In one aspect of the technology, the imaging is done by positron emission tomography (PET), PET/Computed tomography (CT), PET/Magnetic resonance (MR), planar imaging, SPECT imaging, optical imaging, or dye imaging.

Another aspect of the technology is a method of distinguishing between adenocarcinomas and non-adenocarcinomas in a prostate cancer patient comprising the steps of:
(a) assaying a patient's prostate specific membrane antigen (PSMA) expression; (b) determining, from the assay, if the patient's PSMA expression is indicative of prostate cancer adenocarcinoma or non-adenocarcinoma; and (c) administering to the patient: (i) an anti-androgen therapy if the PSMA is indicative of an adenocarcinoma; or (ii) a chemotherapeutic therapy if the PSMA expression is indicative of a non-adenocarcinoma. In one aspect, the PSMA expression is assayed on tissue specimen, such as a prostate biopsy, transurethral resection of the prostate, or prostatectomy tissue. In another aspect, the PSMA expression is assayed on cells present in bodily fluids, such as blood, urine, prostatic fluid, or semen. In another aspect, the PSMA expression is assayed by imaging. In a related aspect, the imaging is conducted by employing any agent capable of specific binding to PSMA, including an antibody, antibody derivative, PSMA ligand, small molecule PSMA binder, PSMA enzyme inhibitor, PSMA-binding peptide, or PSMA-binding aptamer. In a related aspect, the agent is labeled with another agent that allows detection. In a related aspect, the agent that allows detection is Zirconium-89, Technetium-99m, radioiodine, Indium-111, Copper-64, Iodine-124. In a parallel aspect, the agent that allows detection is a dye, such as indocyanine green, cyanine dyes, fluorescent dyes, infrared dyes, or enzymes. In another aspect, the imaging is done by positron emission tomography (PET), PET/Computed tomography (CT), PET/Magnetic resonance (MR), planar imaging, SPECT imaging, optical imaging, or dye imaging.

One aspect of the technology is a method of distinguishing between lethal versus indolent cancer in a prostate cancer patient comprising the steps of: (a) assaying a patient's prostate specific membrane antigen (PSMA) expression; (b) determining, from the assay, if the patient's PSMA expression is indicative of lethal or indolent prostate cancer; and (c) administering to the patient: (i) surgery and/or radiation therapy with or without anti-androgen therapy if the PSMA is indicative of a lethal prostate cancer; or (ii) active surveillance therapy if the PSMA expression is indicative of an indolent prostate cancer. In one aspect, the PSMA expression is assayed on tissue specimen, such as a prostate biopsy, transurethral resection of the prostate, or prostatectomy tissue. In another aspect, the PSMA expression is assayed on cells present in bodily fluids, such as blood, urine, prostatic fluid, or semen. In another aspect, the PSMA expression is assayed by imaging. In a related aspect, the imaging is conducted by employing any agent capable of specific binding to PSMA, such as an antibody, antibody derivative, PSMA ligand, small molecule PSMA binder, PSMA enzyme inhibitor, PSMA-binding peptide, or PSMA-binding aptamer. In a related aspect, the agent is labeled with another agent that allows detection, such as Zirconium-89, Technetium-99m, radioiodine, Indium-111, Copper-64, Iodine-124. In a parallel aspect, the agent that allows detection is a dye, such as indocyanine green, cyanine dyes, fluorescent dyes, infrared dyes, or enzymes. In another aspect, the imaging is done by positron emission tomography (PET), PET/Computed tomography (CT), PET/Magnetic resonance (MR), planar imaging, SPECT imaging, optical imaging, or dye imaging.

While the present invention has been disclosed with reference to certain embodiments, numerous modifications, alterations, and changes to the described embodiments are possible without departing from the sphere and scope of the present invention, as defined in the appended claims. Accordingly, it is intended that the present invention not be limited to the described embodiments, but that it has the full scope defined by the language of the following claims, and equivalents thereof.

Other embodiments of the invention are as described in the following clauses:
1. A method of distinguishing between adenocarcinomas and non-adenocarcinomas in a prostate cancer patient comprising the steps of:
   (a) assaying a patient's prostate specific membrane antigen (PSMA) expression; and
   (b) determining, from the assay, if the patient's PSMA expression is indicative of prostate cancer adenocarcinoma or non-adenocarcinoma.
2. The method of clause 1, wherein the PSMA expression is assayed on tissue specimen.
3. The method of clause 2, wherein the tissue specimen is a prostate biopsy, transurethral resection of the prostate, or prostatectomy tissue.
4. The method of clause 1, wherein the PSMA expression is assayed on bodily fluids.
5. The method of clause 4, wherein the bodily fluid is blood, urine, prostatic fluid, or semen.
6. The method of clause 1, wherein the PSMA expression is assayed by imaging.
7. The method of clause 6, wherein the imaging is conducted by employing any agent capable of specific binding to PSMA.
8. The method of clause 7, wherein the agent is an antibody, antibody derivative, PSMA ligand, small molecule PSMA binder, PSMA enzyme inhibitor, PSMA-binding peptide, or PSMA-binding aptamer.
9. The method of either clause 7 or 8, wherein the agent is labeled with another agent that allows detection.
10. The method of clause 9, wherein the agent that allows detection is Zirconium-89, Technetium-99m, radioiodine, Indium-111, Copper-64, Iodine-124.
11. The method of clause 9, wherein the agent that allows detection is a dye, such as indocyanine green, cyanine dyes, fluorescent dyes, infrared dyes, or enzymes.
12. The method of clause 6, wherein the imaging is done by positron emission tomography (PET), PET/Computed tomography (CT), PET/Magnetic resonance (MR), planar imaging, SPECT imaging, optical imaging, or dye imaging.
13. A method of distinguishing between lethal and indolent prostate cancer in a patient, comprising the steps of:
   (a) assaying a patient's prostate specific membrane antigen (PSMA) expression; and
   (b) determining, from the assay, if the patient's PSMA expression is indicative of lethal or indolent prostate cancer.
14. The method of clause 13, wherein the PSMA expression is assayed on tissue specimen.
15. The method of clause 14, wherein the tissue specimen is a prostate biopsy, transurethral resection of the prostate, or prostatectomy tissue.
16. The method of clause 13, wherein the PSMA expression is assayed on cells present in bodily fluids.
17. The method of clause 16, wherein the bodily fluid is blood, urine, prostatic fluid, or semen.
18. The method of clause 13, wherein the PSMA expression is assayed by imaging.
19. The method of clause 18, wherein the imaging is conducted by employing any agent capable of specific binding to PSMA.
20. The method of clause 19, wherein the agent is an antibody, antibody derivative, PSMA ligand, small molecule PSMA binder, PSMA enzyme inhibitor, PSMA-binding peptide, or PSMA-binding aptamer.
21. The method of either clause 19 or 20, wherein the agent is labeled with another agent that allows detection.
22. The method of clause 21, wherein the agent that allows detection is Zirconium-89, Technetium-99m, radioiodine, Indium-111, Copper-64, Iodine-124.
23. The method of embodiment 21, wherein the agent that allows detection is a dye, such as indocyanine green, cyanine dyes, fluorescent dyes, infrared dyes, or enzymes
24. The method of clause 18, wherein the imaging is done by positron emission tomography (PET), PET/Computed tomography (CT), PET/Magnetic resonance (MR), planar imaging, SPECT imaging, optical imaging, or dye imaging.
25. A method of distinguishing between adenocarcinomas and non-adenocarcinomas in a prostate cancer patient comprising the steps of:
   (a) assaying a patient's prostate specific membrane antigen (PSMA) expression;
   (b) determining, from the assay, if the patient's PSMA expression is indicative of prostate cancer adenocarcinoma or non-adenocarcinoma; and
   (c) administering to the patient:
      (i) an anti-androgen therapy if the PSMA is indicative of an adenocarcinoma; or
      (ii) a chemotherapeutic therapy if the PSMA expression is indicative of a non-adenocarcinoma.
26. The method of clause 25, wherein the PSMA expression is assayed on tissue specimen.
27. The method of clause 26, wherein the tissue specimen is a prostate biopsy, transurethral resection of the prostate, or prostatectomy tissue.
28. The method of clause 25, wherein the PSMA expression is assayed on cells present in bodily fluids.
29. The method of clause 28, wherein the bodily fluid is blood, urine, prostatic fluid, or semen.
30. The method of clause 25, wherein the PSMA expression is assayed by imaging.
31. The method of clause 30, wherein the imaging is conducted by employing any agent capable of specific binding to PSMA.
32. The method of embodiment 31, wherein the agent is an antibody, antibody derivative, PSMA ligand, small molecule PSMA binder, PSMA enzyme inhibitor, PSMA-binding peptide, or PSMA-binding aptamer.
33. The method of either clause 31 or 32, wherein the agent is labeled with another agent that allows detection.
34. The method of clause 33, wherein the agent that allows detection is Zirconium-89, Technetium-99m, radioiodine, Indium-111, Copper-64, Iodine-124.
35. The method of clause 33, wherein the agent that allows detection is a dye, such as indocyanine green, cyanine dyes, fluorescent dyes, infrared dyes, or enzymes.
36. The method of clause 30, wherein the imaging is done by positron emission tomography (PET), PET/Computed tomography (CT), PET/Magnetic resonance (MR), planar imaging, SPECT imaging, optical imaging, or dye imaging.
37. A method of distinguishing between lethal versus indolent cancer in a prostate cancer patient comprising the steps of:
   (a) assaying a patient's prostate specific membrane antigen (PSMA) expression;
   (b) determining, from the assay, if the patient's PSMA expression is indicative of lethal or indolent prostate cancer; and
   (c) administering to the patient:
      (i) surgery and/or radiation therapy with or without anti-androgen therapy if the PSMA is indicative of a lethal prostate cancer; or
      (ii) active surveillance therapy if the PSMA expression is indicative of an indolent prostate cancer.
38. The method of clause t 37, wherein the PSMA expression is assayed on tissue specimen.
39. The method of clause 38, wherein the tissue specimen is a prostate biopsy, transurethral resection of the prostate, or prostatectomy tissue.
40. The method of clause 37, wherein the PSMA expression is assayed on cells present in bodily fluids.
41. The method of clause 40, wherein the bodily fluid is blood, urine, prostatic fluid, or semen.
42. The method of clause 37, wherein the PSMA expression is assayed by imaging.
43. The method of clause 42, wherein the imaging is conducted by employing any agent capable of specific binding to PSMA.
44. The method of clause 43, wherein the agent is an antibody, antibody derivative, PSMA ligand, small molecule PSMA binder, PSMA enzyme inhibitor, PSMA-binding peptide, or PSMA-binding aptamer.
45. The method of either clause 43 or 44, wherein the agent is labeled with another agent that allows detection.
46. The method of clause 45, wherein the agent that allows detection is Zirconium-89, Technetium-99m, radioiodine, Indium-111, Copper-64, Iodine-124.
47. The method of clause 45, wherein the agent that allows detection is a dye, such as indocyanine green, cyanine dyes, fluorescent dyes, infrared dyes, or enzymes.
48. The method of clause 42, wherein the imaging is done by positron emission tomography (PET), PET/Computed tomography (CT), PET/Magnetic resonance (MR), planar imaging, SPECT imaging, optical imaging, or dye imaging.

## Claims

1. A medicament for use in a method of treating prostate cancer, wherein the method comprises assaying a patient's prostate-specific membrane antigen (PSMA) expression, and determining from the assay if the patient's PSMA expression is indicative of a prostate cancer adenocarcinoma or non-adenocarcinoma and administering to the patient the medicament, wherein the medicament is an anti-androgen therapy if PSMA expression is indicative of an adenocarcinoma and wherein the medicament is a chemotherapeutic agent if PSMA expression is indicative of a non-adenocarcinoma.

2. The medicament of claim 1, wherein PSMA expression is assayed by imaging.

3. The medicament of claim 1, wherein the imaging is conducted by employing any agent capable of specific binding to PSMA, wherein preferably, the agent is an antibody, antibody derivative, PSMA ligand, small molecule PSMA binder, PSMA enzyme inhibitor, PSMA-binding peptide, or PSMA-binding aptamer.

4. The medicament of any preceding claim, wherein the imaging is done by positron emission tomography (PET), PET/Computed tomography (CT), PET/Magnetic resonance (MR), planar imaging, SPECT imaging, optical imaging or dye imaging.

5. The medicament of any preceding claim, wherein the non-adenocarcinoma comprises a prostate small cell, neuroendocrine or sarcoma.

6. A method of distinguishing between adenocarcinomas and non-adenocarcinomas in a prostate cancer patient, comprising determining from an assay of a patient's PSMA expression if the patient's PSMA expression is indicative of prostate cancer adenocarcinoma or non-adenocarcinoma.

7. The method of claim 6, wherein the PSMA expression is assayed on a tissue specimen, wherein preferably, the tissue specimen is a prostate biopsy, transurethral resection of the prostate, or prostatectomy tissue.

8. The method of claim 6, wherein the PSMA expression is assayed on bodily fluids, wherein preferably the bodily fluid is blood, urine, prostatic fluid, or semen.

9. The method of claim 6, wherein preferably the PSMA expression is assayed by imaging, wherein preferably, the imaging is conducted by employing an agent capable of specific binding to PSMA.

10. The method of claim 9, wherein the agent is an antibody, antibody derivative, PSMA ligand, small molecule PSMA binder, PSMA enzyme inhibitor, PSMA-binding peptide, or PSMA-binding aptamer.

11. The method of either claim 9 or 10, wherein the agent is labelled with another agent that allows detection.

12. The method of claim 11, wherein the agent that allows detection is Zirconium-89, Technetium-99m, radioiodine, Indium-111, Copper-64 or lodine-124.

13. The method of claim 11, wherein the agent that allows detection is a dye, such as indocyanine green, cyanine dyes, fluorescent dyes, infrared dyes, or enzymes.

14. The method of claim 9, wherein the imaging is done by positron emission tomography (PET), PET/Computed tomography (CT), PET/Magnetic resonance (MR), planar imaging, SPECT imaging, optical imaging or dye imaging.

15. The medicament or method of any preceding claim, wherein the patient's PSMA expression is indicative of a prostate cancer adenocarcinoma if PSMA is expressed, and the patient's PSMA expression is indicative of a prostate cancer non-adenocarcinoma if PSMA is not expressed.
